# EUROPEAN PATENT APPLICATION

(11) **EP 2 537 455 A1**
(43) Date of publication of application: **26.12.2012**
(21) Application number: 11171057.0
(22) Date of filing: 22.06.2011
(51) Int. Cl.: A61B 1/04

(54) **Expandable capsule endoscope and expandable capsule endoscopy system**

(71) Applicant: Chung-Shan Institute of Science and Technology, Armaments, Bureau, Ministry of National Defense, Taoyuan County 325, Chinese Taipei (TW)
(72) Inventor: Yeh, Chuen-Tai, 325 Taoyuan County, Taipei (TW); Lin, Tah-Yeong, 325 Taoyuan Country, Taipei (TW); Wu, Shsien-Ming, 325 Taoyuan County, Taipei (TW)
(74) Representative: Lang, Christian

(57) **Abstract**

The present invention provides an expandable capsule endoscope and an expandable capsule endoscopy system thereof. The expandable capsule endoscope is swallowed to provide the image inspection function of the digestive tract. The expandable capsule endoscope includes an endoscopy function module, an expandable structure and an expansion function module. The endoscopy function module is configured to capture the image outside the expandable capsule endoscope. The expandable structure covers the endoscopy function module. The expansion function module is disposed inside the expandable structure and configured to control the expansion of the expandable structure according to an expansion signal. Therefore, the expandable capsule endoscope can expand itself to a volume to stretch and flatten the wrinkles or folds of the intestine wall so as to facilitate image capturing and maintain the orientation of the expandable capsule endoscope.

## Description

### BACKGROUND OF THE INVENTION

### 1. Field of the invention

The present invention relates generally to a capsule endoscope and a capsule endoscopy system thereof, and more particularly, to an expandable capsule endoscope and an expandable capsule endoscopy system thereof.

### 2. Description of the prior art

In the diagnosis of the digestive diseases, the endoscope is an effective tool widely used. The conventional endoscope has a camera which is disposed on the front side of the endoscopic catheter. The camera, together with the endoscopic catheter, is inserted into the digestive tract through the patient's mouth or anus. The endoscopic catheter has electrical circuits and optical fibers inside thereof. The external part of the endoscope machine can offer the illumination light to the camera through the fiber, while the image captured by the camera is transmitted back to the external machine through the electrical circuit or the fibers as well.

Since the digestive tract has a considerable length and a structure of twists and turns, it is difficult for the endoscope to move along the twisted digestive tract. Most of the endoscopes that are widely used nowadays can only reach the front-end portion of the small intestine through the mouth or to the end portion of the large intestine through the anus, and are unable to move deeply into the middle part of the small intestine. Furthermore, while moving through the digestive tract, the endoscopic catheter will pull and drag the digestive tract and thereby causes great pain for the patients. As a result, the patients are often afraid of the gastrointestinal endoscopic inspection.

To relieve the pain and make it easier to accept the gastrointestinal endoscopic inspection for a patient, the capsule endoscope has therefore been developed in recent year. The capsule endoscope can be swallowed into the human digestive tract without any pain and can move through the digestive tract by the peristalsis of the intestine. The capsule endoscope comprises an image sensor, a wireless transmitter and a battery, which are all integrated into a capsule shell of about 2.6 cm in length and 1.1 cm in diameter. The battery offers the power required by the operation of the image sensor and the wireless transmitter. The image sensor is used to capture the images of the intestinal wall. The captured image will then be converted into the digital information and then transmitted to the external controlling device by the wireless transmitter.

In order to go through the small intestine which has a relatively smaller diameter compared to that of the large intestine, the size of the capsule endoscope has to be designed to be small enough to fit into the diameter of the small intestine. However, designs of this kind are unable to have the capsule endoscopes confined by the loose large intestine wall to keep the orientation in space when traveling through the large intestine with greater diameter than that of the small intestine. Therefore, random rotation of the capsule endoscope often occurs in the large intestine so that tracing of the image would be difficult due to the inconsistent camera direction. Furthermore, because the size of the capsule endoscope is not large enough to fully stretch and flatten the wrinkles and folds of the large intestine wall, the intestinal folds would form a lot of blind spots for the camera, which further increase the difficulty for the inspection of the large intestine.

In view of rising global incidents of the colorectal cancer in recent years, if the difficulties mentioned hereinabove can be overcome to make it possible for the capsule endoscope to perform the inspection of the large intestine, more comfortable inspection ways can be offered to the patients. As a result, the patients would be more willing to accept the gastrointestinal endoscopic inspection, and therefore it would reduce the chances of the development of colorectal malignancy.

### SUMMARY OF THE INVENTION

The objective of the present invention is to provide an expandable capsule endoscope to solve the shortcomings of the prior art.

According to one embodiment of the present invention, the expandable capsule endoscope comprises an endoscopic function module, an expandable structure and an expansion function module. The endoscopic function module is configured to capture an image outside the expandable capsule endoscope. The expandable structure covers outside the endoscopy function module. The expansion function module is disposed inside the expandable structure, and is configured to control the expandable structure to expand according to an expansion signal.

Another objective of the present invention is to provide an expandable capsule endoscopy system to solve the shortcomings of the prior art.

According to one embodiment of the present invention, the expandable capsule endoscopy system comprises an external controlling device and an expandable capsule endoscope. The external controlling device comprises a remote transmitting unit, which is configured to emit an expansion signal wirelessly. The expandable capsule endoscope comprises an endoscopic function module, an expandable structure and an expansion function module. The endoscopic function module is configured to capture an image outside the expandable capsule endoscope. The expandable structure covers outside the endoscopy function module. The expansion function module is configured to wirelessly receive the expansion signal and to control the expandable structure to expand according to the expansion signal.

In conclusion, the capsule endoscope of the present invention can expand the volume thereof according to the remote control signal. When it is determined that the capsule endoscope reaches the large intestine, the external controlling device can emit the expansion signal wirelessly to control the expandable capsule endoscope to expand. By this configuration, the expandable capsule endoscope is able to stretch and flatten the wrinkles and folds of the large intestine wall to capture detailed images and as well prevent the random rotation of the capsule endoscope to keep the camera direction consistent, so as to make it easier to trace the specific position from the image capturing.

The advantage and spirit of the invention may be understood by the following recitations together with the appended drawings.

### BRIEF DESCRIPTION OF THE APPENDED DRAWINGS

**FIG. 1** is a schematic view of the expandable capsule endoscopy system according to one embodiment of the present invention.
**FIG. 2** is a functional block diagram of the expandable capsule endoscopy system according to one embodiment of the present invention.

### DETAILED DESCRIPTION OF THE INVENTION

The present invention provides an expandable capsule endoscope and an expandable capsule endoscopy system thereof for capturing the image of the large intestine wall. The preferred embodiment of the present invention will be described in the following detailed description.

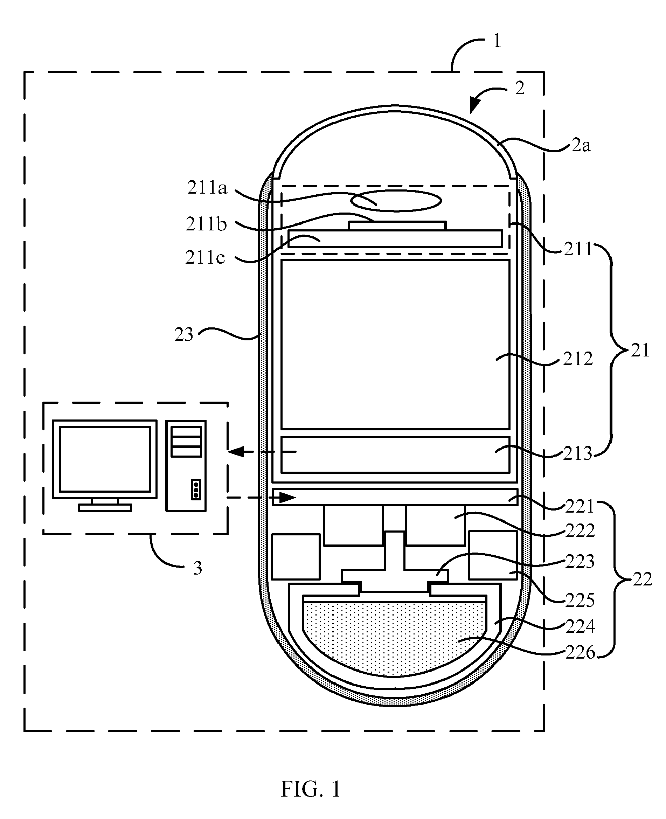

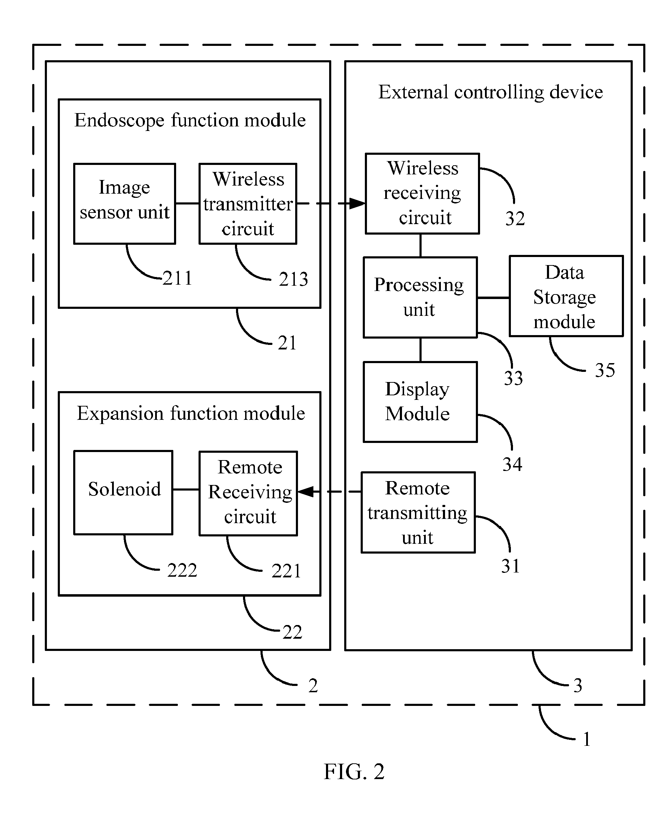

Please refer to **FIG. 1** and **FIG. 2** together. **FIG. 1** illustrates the expandable capsule endoscopy system **1** according to one embodiment of the present invention. **FIG. 2** shows the functional block diagram of the expandable capsule endoscopy system **1** according to one embodiment of the present invention. The expandable capsule endoscopy system **1** comprises an expandable capsule endoscope **2** and an external controlling device **3**. The expandable capsule endoscope **2** mainly comprises an endoscopic function module **21**, an expansion function module **22** and an expandable structure **23**.

The endoscopic function module **21** offers the image capturing function to the expandable capsule endoscope **2** and comprises a sensor unit **211**, a battery **212** and a wireless transmitter circuit **213**. The image sensor unit **211** comprises an optical lens **211a**, a photoelectric sensor **211b** and an image processing circuit **211c**. The front end of the expandable capsule endoscope **2** is a transparent dome **2a** such that the surrounding image can be projected into the expandable capsule endoscope **2**. The optical lens **211a** is disposed inside the front end of the expandable capsule endoscope **2** such that the surrounding image is collected and focused onto the photoelectric sensor **211b**. The photoelectric sensor **211b** converts the image into an electric signal, while the image processing circuit **211c** converts the electric signal into digital image information. Furthermore, the digital image information will then be transmitted to the external controlling device **3** by the wireless transmitter circuit **213**. The battery **212** is adapted to supply the power required to operate all the elements of the expandable capsule endoscope **2**.

The expandable structure **23** is a balloon like structure, which is flexible and hallow, and it has a smooth profile without sharp angle. The material of the expandable structure **23** is of biocompatibility. Furthermore, the expandable structure **23** collapses and covers the outside of the endoscopic function module **21** before expanded, and exposes only the transparent dome **2a** in front of the expandable capsule endoscope **2**. In addition, the expandable structure **23** forms a closed space inside thereof and therefore can be filled with filling substance to enable expansion to an adequate size. With the expanded size, the expandable structure **23** is able to stretch and flatten the wrinkles and folds of the large intestine wall and prevents the random rotation of the expandable capsule endoscope **2** inside the large intestine.

In the practical application, the size of the expandable capsule endoscope **2** is considered according to the difference between the diameters of the small intestine and the large intestine. Generally, the diameter of the small intestine is about 2.5 to 4 cm while the diameters of the blind intestine, the colon and the rectum of the large intestine are about 4 to 6 cm. Therefore, coupled with the consideration of the intestinal contractility, the diameter of the expandable capsule endoscope **2** should be smaller than the smallest diameter of the small intestine before expanding and naturally smaller in diameter than the smallest diameter of the large intestine after expanding.

The expansion function module **22** is disposed inside the expandable structure **23** and comprises a remote receiving circuit **221.** The remote receiving circuit **221** is able to wirelessly receive the expansion signal emitted by the external controlling device **3**. Next, the expansion function module **22** is adapted to activate an expansion mechanism to control the expandable structure **23** to expand. In this embodiment, the expansion mechanism is carried out by controlling the reaction of at least one reacting substance to produce a gas to inflate the expandable structure **23**. For example, the reacting substance of the expansion function module **22** can be either a solid gas-storage material or a compressed gas, and is sealed and stored inside a storage chamber. The storage chamber is disposed with a movable spacer structure. When the remote receiving circuit **221** receives an expansion signal, the movable spacer structure will be controlled to move in order to open the storage chamber accordingly. In the next step, the reacting substance will be controlled to release a non-toxic gas into the expandable structure **23** and therefore expands the expandable structure **23**.

Preferably, the expansion function module **22** of the present embodiment comprises a storage chamber **224**, a first reacting substance **225** and a second reacting substance **226**. Furthermore, a solenoid valve consisting of the solenoid **222** and the and the valve **223** is served as the movable spacer structure. The first reacting substance **225** is disposed outside the storage chamber **224**, while the second reacting substance **226** is sealed and stored inside the storage chamber **224**. Before activating the expansion mechanism, the solenoid valve seals the opening of the storage chamber **224** to isolate the first reacting substance **225** from the second reacting substance **226**. When the expansion function module **22** receives the expansion signal emitted from the external controlling device **3**, the expansion function module **22** will electrify the solenoid **222** to produce the magnetic field to move the valve **223**, so as to connect the spaces in which the first reacting substance **225** and the second reacting substance **226** are stored. By this configuration, the first reacting substance **225** and the second reacting substance **226** can be exposed to each other and react to produce the gas, and therefore inflate the expandable structure **23**.

In practical application, the first reacting substance **225** can be selected from the group consisting carbonate, bicarbonate or a combination thereof while the second reacting substance **226** can be a weak acid solution which is harmless to the human body. When the solenoid valve is opened, the second reacting substance **226** will flow outside the storage chamber **224** and contact the first reacting substance **225**. In the reaction of first reacting substance **225** and the second reacting substance **226**, carbon dioxide will be released gradually and inflates the expandable structure **23** to an adequate size.

The external controlling device **3** comprises a remote transmitting unit **31**, a wireless receiving circuit **32**, a processing unit **33**, a display module **34** and a data storage module **35**. The remote transmitting unit **31** is configured to emit the expansion signal wirelessly. The wireless receiving circuit **32** is configured to receive the digital image information emitted from the wireless transmitter circuit **213** of the endoscopic function module **21**. The processing unit **33** is adapted to store the digital image information into the data storage module **35** for later inspection. Furthermore, the processing unit **33** is also adapted to control the display module **34** to display the digital image information received.

By the above configuration, the inspector can view the image of the digestive tract captured and transmitted by the expandable capsule endoscope **2** in real time. When the inspector sees that the expandable capsule endoscope **2** is entering the large intestine, the remote transmitting unit **31** can be utilized to transmit the expansion signal wirelessly. When the expandable capsule endoscope **2** receives the expansion signal, it will activate the expansion mechanism to inflate the expandable structure **23** to a predetermined volume to stretch and flatten the wrinkles and folds of the large intestine wall. Thereby, the expandable capsule endoscope **2** can easily obtain clear images of the intestine wall, and prevent random rotation to keep a consistent camera direction.

In conclusion, the applicable range can be extended with the expandable capsule endoscopy system **1** and the expandable capsule endoscope **2** of the present invention. In view of the larger amount of lesions in the large intestine than in the small intestine and that the global incidence rate is rising with years, the present invention can reduce the cost, the risk and the pain inflicted in the inspection of large intestine, and it substantially reduces the chances of deterioration in colorectal cancer.

With the example and explanations above, the features and spirits of the invention will be hopefully well described. Those skilled in the art will readily observe that numerous modifications and alterations of the device may be made while retaining the teaching of the invention. Accordingly, the above disclosure should be construed as limited only by the metes and bounds of the appended claims.

## Claims

1. An expandable capsule endoscope, comprising:
an endoscopic function module, configured to capture an image outside the
expandable capsule endoscope;
an expandable structure, covering outside the endoscopy function module;
and
an expansion function module, disposed inside the expandable structure, and
configured to control the expandable structure to expand according to an expansion signal.

2. The expandable capsule endoscope of claim 1, wherein the endoscopic function module comprises an image sensor unit, a battery and a wireless transmitter circuit, the image sensor unit is configured to convert the image into a digital information, the wireless transmitter circuit is configured to transmit the digital information to an external control system, and the battery is adapted to supply a power required by the operation of the expandable capsule endoscope.

3. The expandable capsule endoscope of claim 1, wherein the expansion function module comprises a remote receiving circuit, and the remote receiving circuit is configured to receive the expansion signal wirelessly.

4. The expandable capsule endoscope of claim 1, wherein the expansion function module comprises at least one reacting substance, and is adapted to control the at least one reacting substance to produce a gas to expand the expandable structure.

5. The expandable capsule endoscope of claim 1, wherein the expansion function module comprises a first reacting substance, a second reacting substance and a storage chamber, the storage chamber is adapted to seal and store the second reacting substance, and the expansion function module is adapted to open the storage chamber to enable the reaction between the first reacting substance and the second reacting substance, so as to produce a gas to expand the expandable structure.

6. The expandable capsule endoscope as claimed in claim 5, wherein the expansion function module further comprises a movable spacer structure, disposed on the storage chamber, and the expansion function module controls the movable spacer structure to move to open the chamber.

7. The expandable capsule endoscope of claim 6, wherein the movable spacer structure is a solenoid valve.

8. The expandable capsule endoscope of claim 5, wherein the first reacting substance is in solid form and the second reacting substance is in liquid form.

9. The expandable capsule endoscope of claim 8, wherein the first reacting substance is selected from the group consisting of carbonate, bicarbonate or a combination thereof, the second reacting substance is an acid solution, and the first reacting substance and the second reacting substance are adapted to react with each other to produce carbon dioxide.

10. An expandable capsule endoscopy system, comprising:
an external controlling device, comprising a remote transmitting unit, the
remote transmitting unit being configured to emit an expansion signal wirelessly; and
an expandable capsule endoscope, comprising an endoscopic function
module, an expandable structure and an expansion function module, the endoscopic function module being configured to capture an image outside the expandable capsule endoscope, the expandable structure covering outside the endoscopy function module, and the expansion function module being configured to wirelessly receive the expansion signal and to signal and to control the expandable structure to expand according to the expansion signal.

## Amended claims

### Amended claims in accordance with Rule 137(2) EPC.

**1.** An expandable capsule endoscope (2), comprising:
an endoscopic function module (21), configured to capture an image outside the expandable capsule endoscope (2);
an expandable structure (23), covering outside the endoscopic function module (21); and
an expansion function module (22), disposed inside the expandable structure (23), and configured to control the expandable structure (23) to expand according to an expansion signal.

**2.** The expandable capsule endoscope (2) of claim 1, wherein the endoscopic function module (21) comprises an image sensor unit (211), a battery (212) and a wireless transmitter circuit (213), the image sensor unit (211) is configured to convert the image into a digital information, the wireless transmitter circuit (213) is configured to transmit the digital information to an external control system (3), and the battery (212) is adapted to supply a power required by the operation of the expandable capsule endoscope (2).

**3.** The expandable capsule endoscope (2) of claim 1, wherein the expansion function module (22) comprises a remote receiving circuit (221), and the remote receiving circuit (221) is configured to receive the expansion signal wirelessly.

**4.** The expandable capsule endoscope (2) of claim 1, wherein the expansion function module (22) comprises at least one reacting substance (225), and is adapted to control the at least one reacting substance (225) to produce a gas to expand the expandable structure (23).

**5.** The expandable capsule endoscope (2) of claim 1, wherein the expansion function module (22) comprises a first reacting substance (225), a second reacting substance (226) and a storage chamber (224), the storage chamber (224) is adapted to seal and store the second reacting substance (226), and the expansion function module (22) is adapted to open the storage chamber (224) to enable the reaction between the first reacting substance (225) and the second reacting substance (226), so as to produce a gas to expand the expandable structure (23).

**6.** The expandable capsule endoscope (2) as claimed in claim 5, wherein the expansion function module (22) further comprises a movable spacer structure, disposed on the storage chamber (224), and the expansion function module (22) controls the movable spacer structure to move to open the chamber (224).

**7.** The expandable capsule endoscope (2) of claim 6, wherein the movable spacer structure is a solenoid valve (223).

**8.** The expandable capsule endoscope (2) of claim 5, wherein the first reacting substance (225) is in solid form and the second reacting substance (226) is in liquid form.

**9.** The expandable capsule endoscope (2) of claim 8, wherein the first reacting substance (225) is selected from the group consisting of carbonate, bicarbonate or a combination thereof, the second reacting substance (226) is an acid solution, and the first reacting substance (225) and the second reacting substance (226) are adapted to react with each other to produce carbon dioxide.

**10.** An expandable capsule endoscopy system (1), comprising:
an external controlling device (3), comprising a remote transmitting unit (31), the remote transmitting unit (31) being configured to emit an expansion signal wirelessly; and
an expandable capsule endoscope (2), comprising an endoscopic function module (21), an expandable structure (23) and an expansion function module (22), the endoscopic function module (21) being configured to capture an image outside the expandable capsule endoscope (2), the expandable structure (23) covering outside the endoscopy function module (21), and the expansion function module (22) being configured to wirelessly receive the expansion signal and to control the expandable structure (23) to expand according to the expansion signal.
